Europäisches Patentamt

European Patent Office

Office européen des brevets

(19)

(11) Veröffentlichungsnummer : **0 125 587 B2**

(12) **NEUE EUROPÄISCHE PATENTSCHRIFT**

(45) Veröffentlichungstag der neuen Patentschrift :
14.08.91 Patentblatt 91/33

(51) Int. Cl.⁵ : **C07C 245/06**, C07C 247/16,
G03C 1/52

(21) Anmeldenummer : 84105089.1

(22) Anmeldetag : 05.05.84

(54) Phlegmatisierte Formen explosionsgefährlicher, organischer Diazo- bzw. Azidoverbindungen für strahlungsempfindliche Zusammensetzungen.

(30) Priorität : 17.05.83 DE 3317883

(43) Veröffentlichungstag der Anmeldung :
21.11.84 Patentblatt 84/47

(45) Bekanntmachung des Hinweises auf die
Patenterteilung :
09.12.87 Patentblatt 87/50

(45) Bekanntmachung des Hinweises auf die
Entscheidung über den Einspruch :
14.08.91 Patentblatt 91/33

(84) Benannte Vertragsstaaten :
DE FR GB

(56) Entgegenhaltungen :
DE-A- 2 521 650
DE-A- 3 100 077

(56) Entgegenhaltungen :
DE-A- 3 126 015
DE-A- 3 234 301
DE-B- 1 002 623
DE-B- 1 024 087
DE-B- 1 104 969
DE-C- 647 667
DE-C- 855 117
DE-C- 954 215
US-A- 2 433 515
US-A- 2 566 709
Römpps Chemie Lexicon, 7. Aufl., Stuttgart,
Band 4 (1974) S. 2633

(73) Patentinhaber : CIBA-GEIGY AG
Klybeckstrasse 141
CH-4002 Basel (CH)

(72) Erfinder : Haas, Günther, Dr.
Saarstrasse 109
W-6903 Neckargemünd (DE)

EP 0 125 587 B2

## Beschreibung

Die Erfindung betrifft phlegmatisierte Formen explosionsgefährlicher organischer Diazo- bzw. Azidoverbindungen für strahlungsempfindliche Zusammensetzungen.

Organische Diazo- und Azidoverbindungen -Verbindungen also, die mindestens eine aus zwei Stickstoffatomen bestehende Diazo- bzw. mindestens eine aus drei Stickstoffatomen bestehende Azidogruppe im Molekül enthalten - erfüllen im allgemeinen mindestens eines der Kriterien des Sprengstoffgesetzes der Bundesrepublik Deutschland. Obgleich die meisten dieser Stoffe als Sprengstoffe im eigentlichen Sinn weder vorgesehen noch geeignet sind, unterliegen sie als « explosionsgefährliche Stoffe » bei Herstellung, Handhabung und Transport den Bestimmungen dieses Gesetzes.

Dies trifft insbesondere zu auf eine Reihe bestimmter Diazo- bzw. Azidoverbindungen, wie sie bei der Herstellung von strahlungsempfindlichen Zusammensetzungen in grossen Mengen Verwendung finden. Strahlungsempfindliche Diazo- bzw. Azidoverbindungen enthaltende Zusammensetzungen dienen zur Erzeugung von positiven bzw. negativen Reliefbildern und werden verwendet bei der Herstellung von Druckplatten oder als Photoresists bei der Halbleiterfertigung. Hierbei werden entsprechende Substrate mit den strahlungsempfindlichen Materialien beschichtet, bildmässig mit aktinischer Strahlung belichtet und einem Entwicklungsvorgang unterworfen, wobei dann die positiven bzw. negativen Reliefstrukturen auf dem Substrat erhalten werden.

Für Druckplatten und Positivphotoresists sind seit langem Gemische im Einsatz, die neben einem alkalilöslichen polymeren Bindemittel, das im allgemeinen aus einem Kondensationsprodukt vom Typ der Novolak-Harze besteht, Diazo-Verbindungen vom Typ der o-Chinondiazide als strahlungsempfindliche Komponenten enthalten. Letztere Verbindungen sind normalerweise Veresterungsprodukte der 1 - Oxo-2-diazo-naphthalin-4- oder -5-sulfonsäure mit meist niedermolekularen, phenolische Hydroxylgruppen tragenden Komponenten, wie sie beispielsweise in der DE-PS 938 233 und zahlreichen nachfolgenden Publikationen wie auch der deutschen Patentanmeldung 3 220 816 beschrieben sind. Eine der heute am gebräuchlichsten Verbindungen dieser Art - früher wurden diese häufig als Naphthochinondiazidsulfonylderivate bezeichnet - ist das Veresterungsprodukt aus Naphthochinondiazid-5-sulfonylchlorid mit Trihydroxybenzophenon.

Strahlungsempfindliche Zusammensetzungen für Negativphotoresists bestehen sehr häufig aus teilzyklisiertem Polyisopren und enthalten als Strahlungsvernetzer aromatische Azidoverbindungen. Solche Verbindungen sind beispielsweise in den US-Patentschriften 2 852 379 und 2 940 853 sowie in der deutschen Patentanmeldung 3 234 301 beschrieben. Einer der am häufigsten verwendeten Strahlungsvernetzer für Negativ-Photoresists auf Polyisopren-Basis ist die Bis-azido-Verbindung 2,6-Bis- [(4-azidophenyl) methylen] -4-methylcyclohexanon.

Strahlungsempfindliche Zusammensetzungen für die Erzeugung sowohl von positiven als auch von negativen Reliefbildern kommen als Lösungen in organischen Lösungsmitteln zur Anwendung, welche durch Auflösen der reinen Komponenten in den Lösungsmitteln hergestellt werden.

Diese Herstellung der strahlungsempfindlichen Zusammensetzungen hat den Nachteil, dass hierbei die Handhabung der reinen, die Strahlungsempfindlichkeit bewirkenden Diazo- bzw. Azidoverbindungen erforderlich ist. Da diese Verbindungen als explosionsgefährliche Substanzen angesehen werden, kann ihre Herstellung, Isolierung, Lagerung und Transport sowie Weiterverarbeitung in reiner Form nur unter sehr aufwendigen Vorsichtsmassnahmen und in relativ geringen Mengen erfolgen.

In Anbetracht der heute weit verbreiteten technischen Anwendung dieser Substanzen ist es daher wünschenswert, diese Verbindungen in stabilisierter Form zur Verfügung zu stellen, so dass diese nicht mehr explosionsgefährlich sind und gefährdungsfrei gehandhabt werden können.

Aus dem Bereich der Sprengstoffe ist bekannt, die Empfindlichkeit von Explosivstoffen durch den Zusatz von Phlegmatisierungsmitteln wie etwa Wasser oder organischen Stoffen wie beispielsweise Öl, Paraffin oder Wachs herabzusetzen. Kieselgur ist als einziges mineralisches Phlegmatisierungsmittel speziell nur für das flüssige Nitroglycerin gebräuchlich, wobei dieses in die Hohlräume der porösen Kristallstruktur des Kieselgur aufgenommen wird.

Diese Methoden sind jedoch für die Stabilisierung der genannten Diazo- bzw. Azidoverbindungen nicht geeignet. Da die Eigenschaften von strahlungsempfindlichen Zusammensetzungen, z.B. deren Strahlungsempfindlichkeit, Auflösungsvermögen und Haftfestigkeit, entscheidend von der Reinheit ihrer Einzelkomponenten abhängen, müssen auch diese Verbindungen in hochreiner Form vorliegen. Die quantitative Abtrennung von Zusätzen, wie sie zur Phlegmatisierung von Sprengstoffen bekannt sind, von diesen Diazo- bzw. Azidoverbindungen wäre jedoch nicht oder nur unter erheblichem Trennaufwand erreichbar. Bei diesen Methoden wäre im übrigen ein Abmischen mit den Phlegmatisierungsmitteln durchzuführen, was wiederum einen Umgang mit den reinen explosionsgefährlichen Verbindungen beinhalten würde.

In der DE-OS 3126 015 wird vorgeschlagen, explosionsgefährliche, mehrere Stickstoffatome enthaltende Zwischenprodukte für pharmazeutische Wirksubstanzen in Form ihrer Lösungen in organischen Lösungsmitteln zu stabilisieren. Eine Anwendung dieser Methode auf das vorliegende Problem wäre - unabhängig von der Frage, ob sich hierdurch im vorliegenden Problem ebenfalls eine Phlegmatisierung erreichen liesse - insofern ungünstig, als die Vielzahl der heute als Strahlungssensibilisatoren bzw. Strahlungsvernetzer bei der Herstellung von strahlungsempfindlichen Zusammensetzungen gebräuchlichen explosionsgefährlichen Diazo- bzw. Azidoverbindungen sowie die Vielzahl der für die unterschiedlichsten Anwendungszwecke eingesetzten strahlungsempfindlichen Gemische oft verschiedenster Lösungsmittel bedürfen. Für den jeweiligen unterschiedlichen Verwendungszweck müssten somit die unterschiedlichsten Lösungen bereitgehalten werden.

In der DE-OS 3100 077 wird vorgeschlagen, die Explosionsgefährlichkeit von Naphthochinondiazidsulfonylderivaten dadurch zu beseitigen, dass nur solche Derivate für lichtempfindliche Zusammensetzungen zur Anwendung gelangen, die durch Veresterung von Naphthochinondiazidsulfonylchlorid mit einem Gemisch aus einer niedermolekularen und einer polymeren Verbindung erhalten werden. Dabei soll das Naphthochinondiazidsulfonylchlorid bevorzugt mit der niedermolekularen Komponente zu dem entsprechenden Veresterungsprodukt reagieren. Dieses wird dann als Gemisch mit der polymeren Verbindung isoliert.

Diese Methode ist insofern ungünstig, als die Vielzahl der heute für die verschiedensten Anwendungszwecke verwendeten positiv arbeitenden strahlungsempfindlichen Zusammensetzungen neben dem Naphthochinondiazidsulfonylderivat häufig die verschiedensten polymeren Verbindungen enthalten.

Für den jeweiligen Verwendungszweck müssten somit eine Vielzahl von Mischungen mit den unterschiedlichsten polymeren Verbindungen separat hergestellt werden.

Eine allgemein anwendbare Methode zur Stabilisierung von Diazo- bzw. Azidoverbindungen, wie sie heute zur Herstellung von positiv wie negativ arbeitenden strahlungsempfindlichen Zusammensetzungen benötigt werden, war aus dem Stand der Technik nicht zu ermitteln.

Es bestand daher die Aufgabe, phlegmatisierte Formen explosionsgefährlicher organischer Diazobzw. Azidoverbindungen zu finden, die für die Herstellung von strahlungsempfindlichen Zusammensetzungen geeignet sind.

Diese Aufgabe wurde durch die vorliegende Erfindung gelöst.

Es wurde überraschend gefunden, dass phlegmatisierte Formen explosionsgefährlicher organischer Diazo- bzw. Azidoverbindungen, die unlösliche pulverige anorganische Adsorbentien als Phlegmatisierungsmittel enthalten, keinerlei Explosionsgefährlichkeit aufweisen und sich deshalb in hervorragender Weise für die Herstellung strahlungsempfindlicher Zusammensetzungen eignen.

Gegenstand der Erfindung sind somit phlegmatisierte Formen explosionsgefährlicher organischer Diazo- bzw. Azidoverbindungen für die Verwendung in strahlungsempfindlichen Zusammensetzungen, die unlösliche pulverige anorganische Adsorbentien als Phlegmatisierungsmittel enthalten.

Gegenstand der Erfindung ist weiterhin ein Verfahren zur Herstellung phlegmatisierter Formen organischer Diazo- bzw. Azidoverbindungen für die Verwendung in strahlungsempfindlichen Zusammensetzungen. Hierbei scheidet man diese durch Fällung aus ihren Lösungen in Gegenwart von unlöslichen pulverigen anorganischen Adsorbentien als Phlegmatisierungsmittel ab.

Ebenfalls ist Gegenstand der Erfindung ein Verfahren zur Herstellung strahlungsempfindlicher Zusammensetzungen, die organische Diazo- bzw. Azidoverbindungen enthalten, wobei man deren phlegmatisierte Formen in Lösung mit den weiteren Komponenten der strahlungsempfindlichen Zusammensetzung mischt. Hierbei werden diese mit unlöslichen pulverigen anorganischen Adsorbentien phlegmatisierten Diazo- bzw. Azidoverbindungen in dem Lösungsmittel gelöst und das unlösliche anorganische Material vor oder nach der Mischung mit den weiteren Komponenten der strahlungsempfindlichen Zusammensetzung abgetrennt.

Gegenstand der Erfindung ist darüberhinaus die Verbindung von durch unlösliche pulverige anorganische Adsorbentien phlegmatisierten Diazo- bzw. Azidoverbindungen zur Herstellung strahlungsempfindlicher Zusammensetzungen.

Als erfindungsgemässe Phlegmatisierungsmittel für explosionsgefährliche organische Diazobzw. Azidoverbindungen sind alle in organischen Lösungsmitteln unlöslichen, in pulveriger Form vorliegenden anorganischen Adsorbentien geeignet. Diese können beispielsweise sein Tone, Bleicherden, Aluminiumoxid, Kieselgur, Kieselgel und ähnliche Materialien. Bevorzugt sind Kieselgur, Kieselgel und Aluminiumoxid. Die Teilchengrösse liegt in der Regel zwischen 0,015 und 0,5 mm, ist jedoch unkritisch.

In der phlegmatisierten Form sind diese anorganischen Adsorbentien in einem Anteil von 5-50, vorzugsweise 10-30 Gew.-%, bezogen auf die organische Diazo- bzw. Azidoverbindung, enthalten.

Erfindungsgemäss können mit den genannten Materialien alle für die Herstellung strahlungsempfindlicher Zusammensetzungen gebräuchlichen organischen Diazo- bzw. Azidoverbindungen in eine phlegmatisierte, also nicht mehr explosionsgefährliche Form überführt werden.

Unter Diazoverbindungen sind etwa jene Naphthochinondiazidsulfonylderivate zu verstehen, die durch Veresterung von 1 -Oxo-2-diazonaphthalin-4- oder -5-sulfonsäure bzw. -halogenid mit niedermolekularen phenolische Hydroxylgruppen tragenden Phenolkomponenten erhalten werden. Diese Verbindungen werden als strahlungsempfindliche Komponenten in positiv arbeitenden lichtempfindlichen Zusammensetzungen auf Basis alkalilöslicher Kondensationsprodukte vom Typ der Novolakharze verwendet. Zur erfindungsgemässen Überführung in phlegmatisierte Formen sind jene Derivate bevorzugt, die Veresterungsprodukte von 1-Oxo-2-diazo-naphthalin-5-sulfonsäure mit bis zu 3 Hydroxylgruppen tragenden Benzol-, Benzoesäure-, Benzoesäureester-, Benzoesäureamide-, Arylalkylketon- bzw. Benzophenon-Derivate sowie den isomeren Formen von Kresol darstellen. Besonders bevorzugt sind Veresterungsprodukte mit Phloroglucin, 2,4-Dihydroxybenzophenon, 2,3,4-Trihydroxybenzophenon, Gallussäureestern, Trihydroxyphenylalkylketonen sowie p-Kresol.

Azidoverbindungen sind solche, die eine oder mehrere an einen aromatischen Ring gebundene Azidogruppen in Molekül enthalten. Sie dienen als Strahlungsvernetzer in negativ arbeitenden strahlungsempfindlichen Zusammensetzungen, wie sie zur Erzeugung von Reliefstrukturen Verwendung finden, etwa als Negativphotoresists auf Basis von cyclisiertem Isopren. Besonders bevorzugt zur erfindungsgemässen Überführung in eine phlegmatisierte Form ist die Verbindung 2,6-Bis-[(4-azidophenyl)methylen]-4-methylcyclohexanon.

Das erfindungsgemässe Verfahren zur Herstellung phlegmatisierter Formen solcher in reiner Form explosionsgefährlichen Diazo- bzw. Azidoverbindungen erfolgt in der Weise, dass man diese aus ihren Lösungen durch Fällung in Gegenwart der genannten unlöslichen pulverigen anorganischen Adsorbentien abscheidet, welche man vor der Fällung den Lösungen bzw. dem Fällungsmittel zugesetzt hat. Hierbei stellt man die Diazo- bzw. Azidoverbindung zunächst in an sich bekannter Weise, wie in den eingangs beispielhaft zitierten Publikationen beschrieben, her, jedoch ohne diese in reiner, trockener und somit explosionsgefährlicher Form zu isolieren. Vielmehr löst man die noch wasser- bzw. lösungsmittelfeuchten Produkte in einem der gängigen Lösungsmittel, fügt nun eines der erfindungsgemässen, als Phlegmatisierungsmittel wirkenden anorganischen Adsorbentien zu und scheidet durch Fällung mit einem der als geeignet beschriebenen Fällungsmittel die Diazo- bzw. Azidoverbindung in durch das Adsorbens phlegmatisierte Form ab. Ebenso kann die Abscheidung der Produkte in phlegmatisierter Form in der Art erfolgen, dass die Lösungen der Diazo- bzw. Azidoverbindungen zu einer Mischung aus Phlegmatisierungsmittel und Fällungsmittel zugefügt werden. Das jeweilige Produkt kann dann durch Filtration abgetrennt und problemlos getrocknet werden.

Dieses Verfahren ist sehr einfach durchzuführen und ist in Bezug auf die zugesetzte Menge an Phlegmatisierungsmittel in einem weiten Rahmen unkritisch. Man erreicht eine wirksame Phlegmatisierung, wenn man die anorganischen Adsorbentien in einem Anteil von 5-50 Gew.-%, bezogen auf die Menge an Diazo- bzw. Azidoverbindung, zusetzt. Die günstigsten Ergebnisse erzielt man bei einem Anteil von 10-30 Gew.-%.

Die nach dem erfindungsgemässen Verfahren in eine phlegmatisierte Form überführten Diazo- bzw. Azidoverbindungen zeigen im Gegensatz zu den entsprechenden reinen Verbindungen keinerlei Explosionsgefährlichkeit mehr. Dies wird nach den Vorschriften des Sprengstoffgesetzes mit bestimmten Tests geprüft (s. Ullmann, Enzyklopädie der technischen Chemie, Bd. 21, S. 649-650 und S. 652).

Als explosionsgefährlich werden demnach Stoffe eingestuft, wenn im

a) Fallhammertest bei einer Schlagenergie von 40 Joule (10 kg Fallgewicht, 40 cm Fallhöhe) oder weniger eine Explosion erfolgt oder

b) im Stahlhülsentest bei einem Düsendurchmesser von 2 mm oder mehr Explosion eintritt oder

c) im Reibungstest bei einer Stiftbelastung von 360 N oder darunter Explosion, Entflammung oder Knistern auftritt.

Die erfindungsgemäss phlegmatisierten Formen organischer Diazo- bzw. Azidoverbindungen erfüllen keines der genannten Kriterien für die Explosionsgefährlichkeit. Sie können also problemlos und in beliebiger Menge wie gewöhnliche Chemikalien gehandhabt werden.

Die gemäss der vorliegenden Erfindung phlegmatisierten Formen organischer Diazo- bzw. Azidoverbindungen eignen sich in hervorragender Weise zur Herstellung strahlungsempfindlicher Zusammensetzungen, wie sie zur Erzeugung von positiven bzw. negativen Reliefbildern bei der Herstellung von Druckplatten oder als Photoresists bei der Halbleiterfertigung Verwendung finden. Ihre problemlose Handhabbarkeit aufgrund des Fehlens jeglicher Explosionsgefahr macht sie angesichts des hohen Bedarfs und Verbrauchs an strahlungsempfindlichen Zusammensetzungen besonders wertvoll für die technische Anwendung.

Das erfindungsgemässe Verfahren zur Herstellung strahlungsempfindlicher Zusammensetzungen, die organische Diazo- bzw. Azidoverbindungen enthalten, erfolgt so, dass man die Komponenten in üblicher Weise entsprechend den an sich bekannten Formulierungen in einem organischen Lösungsmittel mischt, wobei man zunächst die mit unlöslichen, pulverigen anorganischen Adsorbentien phlegmatisierten Diazo- bzw. Azidoverbindungen in dem Lösungsmittel löst und dann das unlösliche anorganische Material vor oder nach der Mischung mit den weiteren Komponenten der strahlungsempfindlichen Zusammensetzung abtrennt. Diese Abtrennung kann durch Filtration erfolgen. Das Verfahren zu Herstellung strahlungsempfindlicher Zusammensetzungen gestaltet sich besonders vorteilhaft, da durch die Anwesenheit der unlöslichen anorganischen Adsorbentien die ohnehin erforderliche Filtration der fertigen Lösungen erleichtert wird und daher auf den üblichen Zusatz von Filtrierhilfsmitteln verzichtet werden kann.

Man erhält auf diese Weise Lösungen, die mit den unter Verwendung der reinen explosionsgefährlichen Diazo- bzw. Azidoverbindungen hergestellten in jeder Hinsicht identisch sind und mit denen sich strahlungsempfindliche Beschichtungen entsprechender Qualität erhalten lassen.

Beispiel 1
Herstellung von mit Kieselgur phlegmatisiertem Veresterungsprodukt aus 1
-Oxo-2-diazo-naphthalin-5-sulfonylchlorid und
2,3,4-Trihydroxybenzophenon.

Das Veresterungsprodukt wird nach den Angaben der DE-PS 938 233 hergestellt, jedoch ohne das erhaltene wasserfeuchte Rohprodukt in reiner, trockener Form zu isolieren.

10 g dieses Rohproduktes werden in 40 ml Aceton bei Raumtemperatur gelöst. Diese Lösung wird langsam zu einer intensiv gerührten Suspension von 1 g Kieselgur in 165 ml 1 %iger Salzsäure gegeben. Dabei wird das Veresterungsprodukt abgeschieden und durch Filtration in mit Kieselgur phlegmatisierter Form isoliert. Das Produkt enthält 10,2 Gew.-% Kieselgur, bezogen auf das eingesetzte Veresterungsprodukt.

Analog wurden hergestellt :
mit Kieselgur phlegmatisiertes Veresterungsprodukt aus 1 -Oxo-2-diazo-naphthalin-5-sulfonylchlorid und 2,4- Dihydroxybenzophenon
mit Kieselgur phlegmatisiertes Veresterungsprodukt aus 1 -Oxo-2-diazo-naphthalin-5-sulfonylchlorid und Phloroglucin
mit Kieselgur phlegmatisiertes Veresterungsprodukt aus 1 -Oxo-2-diazo-naphthalin-5-sulfonylchlorid und p-Kresol
mit Kieselgur phlegmatisiertes Veresterungsprodukt aus 1 -Oxo-2-diazo-naphthalin-5-sulfonylchlorid und Ethylgallat.

Beispiel 2

Mit Aluminiumoxid stabilisierte Veresterungsprodukte
Man verfährt wie in Beispiel 1 mit dem Unterschied, dass anstelle von Kieselgur Aluminiumoxid (0,2 g/g gelöster Substanz) verwendet wird.

Beispiel 3

Mit Kieselgel stabilisierte Veresterungsprodukte
Man verfährt wie in Beispiel 1 mit dem Unterschied, dass anstelle von Kieselgur Kieselgel (0,1 g/g gelöste Substanz) verwendet wird.

Beispiel 4

Herstellung einer mit Kieselgur stabilisierten Bisazidoverbindung
10 g rohes, lösungsmittel- bzw. wasserfeuchtes 2,6- Bis[(4-azidophenyl)methylen] -4-methylcyclohexanon, das man gemäss US-P 2 940 853 hergestellt hat, werden unter Rühren bei 50 °C in 100 ml DMF gelöst. Die erhaltene Lösung lässt man langsam zu einer intensiv gerührten Suspension von 2 g Kieselgur in einer Mischung aus 700 ml Wasser und 12 ml konz. Salzsäure zulaufen. Dabei scheidet sich die Bisazidoverbindung in mit Kieselgur phlegmatisierter Form ab und wird durch Filtration isoliert. Gehalt an Kieselgur : 16%.

Zur Prüfung der Explosionsgefährlichkeit wurden die Substanzen gemäss den Vorschriften des Sprengstoffgesetzes mit dem Fallhammertest, dem Stahlhülsentest und dem Reibungstest untersucht. Keine der phlegmatisierten Formen sprach auf diese Tests an, während die reinen Substanzen allein schon im Fallhammertest eine deutlich positive Reaktion zeigten.

Beispiel 5

Herstellung einer lichtempfindlichen Lösung zur Erzeugung von positiven Reliefbildern
38,8 g phlegmatisiertes Produkt gemäss Beispiel 1 werden bei Raumtemperatur in 340 ml Ethoxyethylacetat eingetragen und 30 Minuten bei Raumtemperatur gerührt. Anschliessend werden die ungelösten Anteile abfiltriert.

Im Filtrat werden unter weiterem Rühren bei Raumtemperatur 125 g eines Kresol- Formaldehydharzes (mittleres Molekulargewicht 5500) gelöst. Nach erneuter Filtration über eine Drucknutsche ist die lichtempfindliche Lösung gebrauchsfertig.

Die lichternpfindliche Lösung wird durch Schleuderbeschichten auf an der Oberfläche thermisch oxidierte Siliziumplättchen aufgetragen, das Lösungsmittel im Vacuum entfernt, die erhaltene lichtempfindliche Schicht bildmässig belichtet und mit einer alkalischen Entwicklerlösung entwickelt. Die Eigenschaften der so erhaltenen lichtempfindlichen Schicht und der durch Belichten und Entwickeln erzeugten positiven Reliefbilder entsprechen (hinsichtlich Lichtempfindlichkeit, Auflösungsvermögen, Haftfestigkeit) völlig denen, die erhalten werden, wenn zur Herstellung der lichtempfindlichen Lösung anstelle des stabilisierten Derivates 35 g reines, getrocknetes Veresterungsprodukt eingesetzt werden.

## Patentansprüche

1. Phlegmatisierte Formen explosionsgefähr licher feste organischer Diazo- bzw. Azidoverbindungen für die Verwendung in strahlungsempfindlichen Zusammensetzungen, dadurch gekennzeichnet, dass diese unlösliche, pulverige anorganische Adsorbentien als Phlegmatisierungsmittel enthalten.

2. Phlegmatisierte Formen nach Anspruch 1, dadurch gekennzeichnet, dass diese, bezogen auf die organischen Diazo- bzw. Azidoverbindungen, 5-50, vorzugsweise 10-30 Gew.-% an Phlegmatisierungsmittel enthalten.

3. Verfahren zur Herstellung phlegmatisierter Formen feste organischer Diazo- bzw. Azidoverbindungen für die Verwendung in strahlungsempfindlichen Zusammensetzungen, dadurch gekennzeichnet, dass man diese aus ihren Lösungen in Gegenwart von unlöslichen, pulverigen anorganischen Adsorbentien als Phlematisierungsmittel abscheidet.

4. Verfahren nach Anspruch 3, dadurch gekennzeichnet, dass man, bezogen auf die organische Diazo- bzw- Azidoverbindung, 5-50, vorzugsweise 10-30 Gew.-% an unlöslichen, pulverigen anorganischen Adsorbentien zusetzt.

5. Verwendung von unlöslichen, pulverigen anorganischen Adsorbentien zur Phlegmatisierung von explosionsgefährlichen feste organischen Diazo- bzw. Azidoverbindungen bei der Herstellung strahlungsempfindlicher Zusammensetzungen.

6. Verfahren zur Herstellung strahlugsempfindlicher Zusammensetzungen, die feste organische Diazo- bzw. Azidoverbindungen enthalten, wobei man die Komponenten in einem organischen Lösungsmittel mischt, dadurch gekennzeichnet, dass man mit unlöslichen, pulverigen anorganischen Adsorbentien phlegmatisierte Diazo- bzw. Azidoverbindungen in dem Lösungsmittel löst und das unlösliche anorganische Material vor oder nach der Mischung mit den weiteren Komponenten der strahlungsempfindlichen Zusammensetzung abtrennt.

7. Verwendung von durch unlöslichen pulverige anorganischen Adsorbentien phlegmatisierten feste organische Diazo- bzw. Azidoverbindungen zur Herstellung strahlungsempfindlicher Zusammensetzungen.

## Claims

1. Desensitised forms of explosive solid organic diazo or azido compounds for use in radiation-sensitive compositions, characterised in that these contain insoluble, pulverulent inorganic adsorbents as desensitising agents.

2. Desensitised forms according to Claim 1, characterised in that these contain 5 - 50% by weight, preferably 10 - 30% by weight, of desensitising agents, based on the organic diazo or azido compounds.

3. Process for the preparation of desensitised forms of solid organic diazo or azido compounds for use in radiation-sensitive compositions, characterised in that these are deposited from their solutions in the presence of insoluble, pulverulent inorganic adsorbents as desensitising agents.

4. Process according to Claim 3, characterised in that 5 - 50% by weight, preferably 10 - 30% by weight, based on the organic diazo or azido compound, of insoluble, pulverulent inorganic adsorbents is added.

5. Use of insoluble, pulverulent inorganic adsorbents for desensitising explosive solid organic diazo or azido compounds in the preparation of radiation-sensitive compositions.

6. Process for the preparation of radiation-sensitive compositions which contain solid organic diazo or azido compounds, the components being mixed in an organic solvent, characterised in that diazo or azido compounds desensitised with insoluble, pulverulent inorganic adsorbents are dissolved in the solvent and the insoluble inorganic material is removed before or after mixing with the other components of the radiation-sensitive composition.

7. Use of solid organic diazo or azido compounds desensitised by insoluble, pulverulent inorganic adsorbents for the preparation of radiation-sensitive compositions.

## Revendications

1. Formes phlegmatisées de composés organiques solides explosibles du type diazoïque ou azide conçues pour l'utilisation dans des compositions sensibles aux rayonnements, caractérisées en ce qu'elles contiennent en tant qu'agents phlegmatisants des adsorbants minéraux pulvérulents insolubles.

2. Formes phlegmatisées selon la revendication 1, caractérisées en ce qu'elles contiennent de 5 à 50, de préférence de 10 à 30% en poids, d'agent phlegmatisant par rapport aux composés organiques du type diazoïque ou azide.

3. Procédé de préparation de formes phlegmatisées de composés organiques solides du type diazoïque ou azide, conçues pour l'utilisation dans des compositions sensibles aux rayonnements, caractérisé en ce que l'on sépare ces composés de leurs solutions en présence d'agents adsorbants minéraux pulvérulents insolubles qui servent d'agents phlegmatisants.

4. Procédé selon la revendication 3, caractérisé en ce que l'on ajoute de 5 à 50, de préférence de 10 à 30% en poids d'adsorbants minéraux pulvérulents insolubles par rapport au composé organique du type diazoïque ou azide.

5. Utilisation d'adsorbants minéraux pulvérulents insolubles pour la phlegmatisation de composés orgaques solides explosibles du type diazoïque ou azide, à la préparation de compositions sensibles aux rayonnements.

6. Procédé de préparation de compositions sensibles aux rayonnements contenant des composés organiques soljdes du type diazoïque ou azide, dans lequel on mélange les composants dans un solvant organique, caractérisé en ce que l'on dissout dans le solvant les diazoïques ou azides phlegmatisés par des adsorbants minéraux pulvérulents insolubles et on sépare la matière minérale insoluble avant ou après le mélange avec les autres composants de la composition sensible aux rayonnements.

7. Utilisation de composés organiques solides du type diazoïque ou azide, phlegmatisés par des adsorbants minéraux ou pulvérulents insolubles, pour la préparation de compositions sensibles aux rayonnements.